Europäisches Patentamt

European Patent Office

Office européen des brevets

(19)

(11) Veröffentlichungsnummer: **0 004 021**
**B1**

(12) **EUROPÄISCHE PATENTSCHRIFT**

(21) Anmeldenummer: **79100576.2**

(22) Anmeldetag: **26.02.79**

(51) Int. Cl.³: **C 07 C  69/96,**
**C 07 C  68/02, C 08 K  5/10**

(54) Verfahren zur Herstellung perhalogenierter Diarylcarbonate und die Verwendung der so erhaltenen Carbonate zur Flammschutzausrüstung von Kunststoffen

(30) Priorität: **10.03.78 DE 2810461**

(43) Veröffentlichungstag der Anmeldung:
**19.09.79 Patentblatt 79/19**

(45) Bekanntmachung des Hinweises auf die Patenterteilung:
**29.10.80 Patentblatt 80/22**

(84) Benannte Vertragsstaaten:
**DE FR GB IT NL**

(56) Entgegenhaltungen:
**DE - A - 1 056 141**
**DE - A - 2 804 227**

(73) Patentinhaber: **BAYER AG**
**Zentralbereich Patente, Marken und Lizenzen**
**D - 5090 Leverkusen 1, Bayerwerk (DE)**

(72) Erfinder: **Alewelt, Wolfgang, Dr.**
**Bodelschwinghstrasse 32**
**D - 4150 Krefeld 1 (DE)**
**Margotte, Dieter, Dr.**
**Wedelstrasse 48**
**D - 4150 Krefeld 1 (DE)**
**Vernaleken, Hugo, Dr.**
**Kreuzbergstrasse 147**
**D - 4150 Krefeld (DE)**

Courier Press, Leamington Spa, England.

## Verfahren zur Herstellung perhalogenierter Diarylcarbonate und die Verwendung der so erhaltenen Carbonate zur Flammschutzausrüstung von Kunststoffen

Gegenstand der Erfindung ist ein Verfahren zur Herstellung von perhalogenierten Diarylcarbonaten nach dem Phasengrenzflächenverfahren durch Umsetzung von wäßrigen Alkalisalzlösungen perhalogenierter Phenole mit Phosgen in Gegenwart eines mit Wasser nicht mischbaren Lösungsmittels.

Es ist bekannt, aromatische Monohydroxyverbindungen mit Phosgen in Gegenwart quaternärer Ammoniumsalze wie z.B. Tetramethylammoniumjodid bei Reaktionstemperaturen oberhalb 150°C zu Diarylcarbonaten umzusetzen. Das Verfahren kann vereinfacht und die Ausbeute an Diarylcarbonaten verbessert werden, wenn die Umsetzung der wäßrigen Alkalisalze von aromatischen Monohydroxyverbindungen mit Phosgen unter Zugabe von mit Wasser nicht mischbaren Lösungsmitteln und unter heftigem Rühren bei 25°C erfolgt (Deutsche Patentschriften 1 056 141 und 1 101 386). Bei dem erstgenannten lösungsmittelfreien Verfahren sind verhältnismäßig lange Reaktionszeiten notwendig, um befriedigende Ausbeuten zu erzielen. Bei der Umsetzung in Gegenwart von Lösungsmitteln muß das Reaktionsprodukt aus dem Lösungsmittel durch Abdampfen isoliert und durch Destillation gereinigt werden.

Weiter ist bekannt, Decarbromdiphenylcarbonat durch Umsetzung von Pentabromophenol mit Phosgen in Gegenwart von Pyridin als Säurefänger herzustellen (USP 3 382 207). Hierbei muß das Reaktionsprodukt von Pyridinhydrochlorid und nicht umgesetztem Ausgangsmaterial und dem als Zwischenprodukt auftretenden Chlorameisensäureester nach einem umständlichen Reinigungsverfahren abgetrennt werden.

Es ist daher die Aufgabe der vorliegenden Erfindung, perhalogenierte Diarylcarbonate nach einem einfachen wirtschaftlichen Verfahren in reiner Form und hoher Ausbeute ohne die genannten Schwierigkeiten herzustellen.

Überraschenderweise gelingt dies durch Umsetzung der wäßrigen Alkalisalzlösungen perhalogenierter Phenole mit Phosgen in Gegenwart von mit Wasser nicht mischbaren organischen Lösungsmitteln, wobei das Lösungsmittel so gewählt wird, daß der als Zwischenprodukt auftretende Chlorameisensäureester gelöst bleibt und das entstehende Diarylcarbonat unlöslich ist. Die perhalogenierten Diarylcarbonate fallen dann während der Reaktion aus und können nach Beendigung der Reaktion durch Abfiltrieren und Waschen mit dem Lösungsmittel in reiner Form isoliert werden. Geringe Mengen nicht umgesetzten Phenols und die anorganischen Salze bleiben in der wäßrigen Alkalihydroxidlösung, nicht umgesetzter Chlorameisensäureester im organischen Lösungsmittel gelöst. Die Reaktion wird durch tert. Amine oder quaternäre Ammoniumverbindungen katalysiert.

Geeignete perhalogenierte Phenole für die Umsetzung sind z.B. Pentafluorphenol, Pentachlorphenol oder Pentabromphenol.

Besonders gute Ausbeuten an perhalogeniertem Diarylcarbonat erhält man, wenn die Umsetzung der wäßrigen Alkaliphenolatlösungen mit Phosgen bei pH-Werten von 7—11, bevorzugt 8—10, bei Temperaturen zwischen 10—90°C, bevorzugt 20—60°C durchgeführt wird. Nach der Phosgenierung läßt man in einer zweiten Reaktionsstufe zur Vervollständigung der Reaktion bei pH 10—13 in Gegenwart eines tert. Amins oder einer quaternären Ammoniumsalzes nachreagieren. Hierbei werden die in der ersten Reaktionsstufe in großer Menge gebildeten Chlorameisensäureester zum Perhalogendiarylcarbonat umgesetzt. Die gewünschten pH-Werte werden durch Zugabe von wäßrigen Alkalihydroxidlösungen, bevorzugt 20—50%iger Natron- oder Kalilauge eingestellt. Der Katalysator kann auch bereits vor der Phosgenierung zugegeben werden, wobei aber mit einer geringfügig höheren Verseifung des Phosgens durch das wäßrige Alkalyhydroxid gerechnet werden muß. Für die Umsetzung wird vorzugsweise ein Phosgenüberschuß von 1,05—2 Mol pro 2 Mol Perhalogenphenol, besonders bevorzugt 1,1—1,5 Mol-Phosgen pro 2 Mol Perhalogenphenol verwendet.

Pro Liter wäßrige Alkalihydroxidlösung werden vorzugsweise 0,5—3 Mol Phenol eingesetzt. Die Menge organischen Lösungsmittels beträgt vorzugsweise 0,5—5 kg/kg perhalogeniertes Diarylcarbonat. Geeignete organische Lösungsmittel für das erfindungsgemäße Verfahren sind chlorierte aliphatische Kohlenwasserstoffe wie z.B. Methylenchlorid, Chloroform, Tetrachlorkohlenstoff, Dichlorethan und Trichlorethan aromatische Kohlenwasserstoffe wie z.B. Benzol, Toluol und Xylol und chlorierte aromatische Kohlenwasserstoffe wie z.B. Chlorbenzol.

Die Katalysatorkonzentration kann in weiten Bereichen variiert werden. Im allgemeinen verwendet man pro Mol eingesetztes Perhalogenphenol 0,001—0,1 Mol und vorzugsweise 0,005—0,05 Mol des tert. Amins oder der quaternären Ammoniumverbindung.

Geeignete Katalysatoren sind aliphatische, araliphatische, cycloaliphatische und heterocyclische tertiäre Amine und die entsprechenden quaternären Ammoniumverbindungen. Beispielhaft seien genannt: Triethylamin, Tri-n-butylamin, N,N-Dimethyl-cyclohexylamin, N-ethyl-piperidin, Tetraethylammoniumchlorid und Triethylbenzylammoniumchlorid. Bevorzugte Katalysatoren sind die tertiären Amine, insbesondere Triethylamin und N-Ethylpiperidin.

Während der gesamten Reaktion muß für intensive Durchmischung gesorgt werden. Dies kann man z.B. durch hochtourige Rühraggregate erreichen.

Wie bereits ausgeführt, wird nach der bevorzugten Ausführungsform des Verfahrens die Umsetzung in zwei Stufen durchgeführt. Während der Phosgenierung wird bei pH 7—11 in 5—60 min, bevorzugt 5—30 min, zunächst der als Zwischenprodukt auftretende Chlorameisensäureester der Perhalogenphenols hergestellt, der unter den gewählten Reaktionsbedingungen nur unvollständig zum Diarylcarbonat weiterreagiert. In der ersten Stufe werden nur geringe Mengen Phosgen verseift, obwohl die Reaktion des Phosgens mit dem Phenol auf Grund sterischer Hinderung durch die o-ständigen Halogengruppen erschwert ist. In der zweiten Stufe werden die Chlorameisensäureester nach Katalysatorzugabe in 5—60 min., bevorzugt 10—30 min, bei pH-Werten von 10—13 zum Diarylcarbonat umgesetzt.

Nach dem erfindungsgemäßen Verfahren werden die perhalogenierten Diarylcarbonate in nahezu quantitativer Ausbeute erhalten. Sie fallen als feine weiße Kristalle an, die nach bekannten Verfahren, z.B. durch Filtration, isoliert werden können. Zur Reinigung werden die Produkte mit dem für die Reaktion verwendeten organischen Lösungsmittel, verdünnter wäßriger Alkalihydroxydlösung und anschließend mit Wasser gewaschen. Eine weitere Reinigung ist im allgemeinen nicht erforderlich.

Das erfindungsgemäße Verfahren wird so durchgeführt, daß in dem Zweiphasengemisch aus verdünnter wäßriger Alkalihydroxidlösung und mit Wasser nicht mischbarem Lösungsmittel nicht umgesetztes Ausgangsmaterial bzw. nicht abreagiertes Zwischenprodukt gelöst bleibt, während das Reaktionsprodukt als nicht lösliches Material ausfällt. Durch diese Reaktionsführung gelingt es, perhalogenierte Diarylcarbonate in sehr reiner Form zu erhalten.

Die perhalogenierten Diarylcarbonate können als Flammschutzmittel für Kunststoffe z.B. für Polycarbonate, Polyamide, Polyester, Polypropylen oder Acrylnitrilstyrol-Copolymerisate verwendet werden. Die folgenden Beispiele sollen das erfindungsgemäße Verfahren erläutern. Die angegebenen Teile sind Gewichtsteile.

### Beispiel 1

97,5 Teile Pentabromphenol werden in 270 Teilen Wasser in einem Dreihalskolben suspendiert, der mit Rührer, Tropftrichter, Gaseinleitungs- und Ableitungsrohr versehen ist. Der Luftsauerstoff wird anschließend aus der Reaktionsmischung entfernt, indem 15 Minuten Stickstoff unter Rühren durch die Reaktionsmischung geleitet wird. Dann werden 18 Teile 45%iger Natronlauge und 210 Teile Chlorbenzol zugegeben. Die Mischung wird auf 25°C gekühlt. Bei dieser Temperatur werden 15 Teile Phosgen bei pH 9—10 in 10 min eingeleitet. Während des Phosgeneinleitens werden 10 Teile 45%iger Natronlauge zur Aufrechterhaltung des pH-Wertes zugetropft. Nach dem Phosgeneinleiten werden 0,8 Teile N-Ethylpiperidin und zur Erhöhung des pH-Wertes auf 12—13, 7,5 Teile 45%iger Natronlauge zugegeben. Zur Vervollständigung der Reaktion läßt man 30 min. nachreagieren.

Zur Aufarbeitung wird vom ausgefallenen Reaktionsprodukt abfiltriert und nacheinander mit Chlorbenzol und verdünnter Natronlauge gewaschen. Anschließend wird mit Wasser elektrolytfrei gewaschen. Das Produkt wird bei 120°C im Wasserstrahlvakuum getrocknet.

Ausbeute: 98% der Theorie, bezogen auf eingesetztes Pentabromphenol. Der Gehalt an verseifbarem Chlor aus nicht umgesetztem Chlorameisensäurepentabromphenylester beträgt <2 ppm.

### Beispiel 2

Beispiel 1 wird wiederholt, jedoch werden 53,5 Teile Pentachlorphenol statt Pentabromphenol eingesetzt.

Ausbeute: 83% der Theorie, bezogen auf eingesetztes Pentachlorphenol. Der Gehalt an verseifbarem Chlor beträgt <2 ppm. Die Ausbeute läßt sich erhöhen durch Eindampfen des Lösungsmittels Chlorbenzol. Dieses Produkt ist aber mit geringen Mengen nicht umgesetzten Chlorameisensäurephenylester verunreinigt. Es ist darum günstiger, dieser Lösungsmittel für weitere Ansätze zu verwenden. Die Ausbeute ist dann nahezu quantitativ.

### Beispiel 3

Beispiel 1 wird wiederholt, jedoch werden 180 Teile Methylenchlorid statt Chlorbenzol verwendet und als Katalysator Triethylamin statt N-Ethylpiperidin verwendet.

Ausbeute: 93% der Theorie, bezogen auf eingesetztes Pentabromphenol. Gehalt an verseifbarem Chlor: <2 ppm. Durch Eindampfen des Lösungsmittels kann wie in Beispiel 2 weiteres Produkt erhalten werden.

### Beispiel 4

Beispiel 1 wird wiederholt, jedoch wird die Reaktion bei 60°C durchgeführt und Triethylamin anstelle von N-Ethylpiperidin verwendet.

Ausbeute: 92% der Theorie, bezogen auf eingesetztes Pentabromphenol. Gehalt an verseifbarem Chlor: <2 ppm. Durch Eindampfen des Lösungsmittels kann wie in Beispiel 2 weiteres Produkt erhalten werden.

### Patentansprüche

1. Verfahren zur Herstellung perhalogenierter Diarylcarbonate durch Umsetzung von Phosgen mit einem Alkalisalz eines perhalogenierten Phenols in einem intensiv durchmischten zweiphasigen Gemisch aus wäßriger Alkalisalzlösung des perhalogenierten Phenols und einem mit Wasser nicht mischbaren organischen Lösungsmittel, dadurch, gekennzeichnet, daß ein Lösungsmittel verwendet wird, in dem der als Zwischenprodukt auftretenden Chlorameisensäureperhalogenphenylester gelöst bleibt und das perhalogenierte Diarylcarbonat unlöslich ist und als Katalysator ein tertiäres Amin oder eine quaternäre Ammoniumverbindung verwendet wird.

2. Verfahren nach Anspruch 1, dadurch gekennzeichnet, daß chlorierte aliphatische Kohlenwasserstoffe, aromatische oder chlorierte aromatische Kohlenwasserstoffe als Lösungsmittel eingesetzt werden.

3. Verfahren nach Anspruch 1 und 2, dadurch gekennzeichnet, daß Methylenchlorid oder Chlorbenzol als Lösungsmittel eingesetzt wird.

4. Verfahren nach Anspruch 1—3, dadurch gekennzeichnet, daß pro kg perhalogenierten Diarylcarbonat 0,5—5 kg organisches Lösungsmittel verwendet werden.

5. Verfahren nach Anspruch 1, dadurch gekennzeichnet, daß die Reaktion in Gegenwart von 0,001—0,1 Mol, pro Mol eingesetzten Perhalogenphenols eines tertiären Amins oder einer quaternären Ammoniumverbindung als Katalysator durchgeführt wird.

6. Verfahren nach Anspruch 5, dadurch gekennzeichnet, daß als Katalysator Triethylamin oder N-Ethylpiperidin verwendet wird.

7. Verfahren nach Anspruch 1, dadurch gekennzeichnet, daß als perhalogenierte Phenole Pentafluorphenol, Pentachlorphenol oder Pentabromphenol eingesetzt werden.

8. Verwendung der nach Anspruch 1—7 erhaltenen perhalogenierten Diarylcarbonate zur Flammschutzausrüstung von Kunststoffen.

**Revendications**

1. Procédé pour la préparation de carbonates de diaryle perhalogénés par réaction du phosgène avec un sel alcalin d'un phénol perhalogéné dans un mélange à deux phases, soumis à mélange intensif consistant en une solution aqueuse d'un sel alcalin du phénol perhalogéné et un solvant organique non miscible à l'eau, ce procédé se caractérisant en ce que l'on utilise un solvant dans lequel le chloroformiate de perhalogénophényle formé en produit intermédiaire reste dissous et le carbonate de diaryle perhalogéné est insoluble et en ce que l'on utilise comme catalyseur une amine tertiaire ou un composé d'ammonium quaternaire.

2. Procédé selon la revendication 1, caractérisé en ce que l'on utilise comme solvants des hydrocarbures aliphatiques chlorés, des hydrocarbures aromatiques ou des hydrocarbures aromatiques chlorés.

3. Procédé selon les revendications 1 et 2, caractérisé en ce que l'on utilise comme solvant le chlorure de méthylène ou le chlorobenzène.

4. Procédé selon les revendications 1 à 3, caractérisé en ce que l'on utilise de 0,5 à 5 kg de solvant organique par kg de carbonate de diaryle perhalogéné.

5. Procédé selon la revendication 1, caractérisé en ce que l'on effectue la réaction en présence de 0,001 à 0,1 mole d'une amine tertiaire ou d'un composé d'ammonium quaternaire, en tant que catalyseur, pour 1 mole de perhalogénophénol mis en oeuvre.

6. Procédé selon la revendication 5 caractérisé en ce que l'on utilise comme catalyseur la triéthylamine ou la N-éthyl-pipéridine.

7. Procédé selon la revendication 1, caractérisé en ce que l'on utilise en tant que phénol perhalogéné le pentafluorophénol, le pentachlorophénol ou le pentabromophénol.

8. Utilisation des carbonates de diaryle perhalogénés obtenus selon les revendications 1 à 7, pour l'ignifugation de résines synthétiques.

**Claims**

1. Process for the preparation of perhalogenated diaryl carbonates by reacting phosgene with an alkali metal salt of a perhalogenated phenol in an intensively mixed two-phase mixture of an aqueous alkali metal salt solution of the perhalogenated phenol and a water-immiscible organic solvent, characterised in that a solvent is used in which the chloroformic acid perhalogen phenyl ester forming as the intermediate product remains dissolved and the perhalogenated diaryl carbonate is insoluble and a tertiary amine or a quaternary ammonium compound is used as catalyst.

2. Process according to Claim 1, characterised in that chlorinated aliphatic hydrocarbons, aromatic or chlorinated aromatic hydrocarbons are used as the solvent.

3. Process according to Claims 1 and 2, characterised in that the solvent used is methylene chloride or chlorobenzene.

4. Process according to Claims 1 to 3, characterised in that 0.5 to 5 kg of organic solvent are used per kg of perhalogenated diaryl carbonate.

5. Process according to Claim 1, characterised in that the reaction is conducted in the presence of 0.001 to 0.1 mole, per mole of perhalogenated phenol used, of a tertiary amine or a quaternary ammonium compound, as catalyst.

6. Process according to Claim 5, characterised in that triethylamine or N-ethylpiperidine is used as catalyst.

7. Process according to Claim 1, characterised in that pentafluorophenyl, pentachlorophenol or pentabromophenol are used as the perhalogenated phenols.

8. Use of the perhalogenated diaryl carbonates obtained according to Claims 1 to 7 for the flame-proofing of plastics.